# EUROPÄISCHE PATENTANMELDUNG

(11) **EP 3 255 033 A1**
(43) Veröffentlichungstag der Anmeldung: **13.12.2017**
(21) Anmeldenummer: 16173915.6
(22) Anmeldetag: 10.06.2016
(51) Int. Cl.: C07C 209/88, C07C 211/36

(54) **DISKRIMINIERUNG VON CIS- UND TRANS-1,3-DIAMINOCYCLOHEXANEN**

(71) Anmelder: BASF SE, 67056 Ludwigshafen am Rhein (DE)
(72) Erfinder: INGRAM, Thomas, 68163 Mannheim (DE); ERNST, Martin, 69121 Heidelberg (DE); PANCHENKO, Alexander, 67071 Ludwigshafen (DE)
(74) Vertreter: Reitstötter Kinzebach

(57) **Zusammenfassung**

Bei einem Verfahren zur Diskriminierung von *cis-* und *trans*-1,3-Diaminocyclohexanen, setzt man ein Gemisch von *cis-* und *trans*-1,3-Diaminocyclohexanen mit Kohlendioxid oder einem reaktiven Kohlensäurederivat um und erhält selektiv den Harnstoff des *cis-*1,3-Diaminocyclohexans, der durch ein mechanisches Trennverfahren abgetrennt werden kann. 4-Methylcyclohexan-1,3-diamin und/oder 2-Methylcyclohexan-1,3-diamin mit einem *trans*-Gehalt von 99 Mol-% oder mehr sind auf diese Weise erhältlich.

## Beschreibung

Die vorliegende Erfindung betrifft ein Verfahren zur Diskriminierung von *cis-* und *trans-*1,3-Diaminocyclohexanen, und insbesondere zur Diskriminierung und Trennung von *cis-* und *trans*-4-Methylcyclohexan-1,3-diamin bzw. *cis-* und *trans*-2-Methylcyclohexan-1,3-diamin.

1,3-Diaminocyclohexane sind beispielsweise durch Hydrierung von 1,3-Phenylen-diaminen zugänglich. Ein derartiges Verfahren ist in der US 6,075,167 beschrieben. Hierbei wird ein stereoisomeres Gemisch von *cis-* und *trans*-1,3-Diaminocyclohexanen in unterschiedlichen Anteilen erhalten. Für bestimmte Anwendungen ist es erforderlich, über die reinen *cis-* bzw. *trans*-Stereoisomere zu verfügen. Da die physikalischen Eigenschaften der Stereoisomere sehr ähnlich sind, ist eine Trennung, z. B. durch fraktionierte Destillation, sehr aufwendig. Eine vollständige Auftrennung ist mit konventionellen Methoden praktisch unmöglich.

Green Chem., 2007, 9, 158-161 beschreibt die Cs⁺-katalysierte Umsetzung von CO₂ und Aminen. Green Chem., 2008, 10, 465-469 beschreibt die durch ionische Flüssigkeiten katalysierte Synthese disubstituierter Harnstoffe aus Aminen und CO₂.

Gemäß Phys. Chem. Chem. Phys., 2012, 14, 464-468 können Polyharnstoffe aus Diaminen und CO₂ in Abwesenheit von Katalysatoren und Lösungsmitteln erhalten werden.

Der Erfindung liegt die Aufgabe zugrunde, ein Verfahren zur Diskriminierung von *cis-*und *trans*-1,3-Diaminocyclohexanen anzugeben, das einfach, wirtschaftlich und effizient ist. Der Erfindung liegt weiterhin die Aufgabe zugrunde, 4-Methylcyclohexan-1,3-diamin, 2-Methylcyclohexan-1,3-diamin oder Gemische davon bereitzustellen, worin die Aminogruppen bezüglich der Cyclohexan-Ringebene im Wesentlichen ausschließlich *trans* angeordnet sind.

Die Aufgabe wird gelöst durch ein Verfahren zur Diskriminierung von *cis-* und *trans-*1,3-Diaminocyclohexanen, wobei man ein Gemisch von *cis-* und *trans*-1,3-Diamino-cyclohexanen mit Kohlendioxid oder einem reaktiven Kohlensäurederivat umsetzt und selektiv den Harnstoff des *cis-*1,3-Diaminocyclohexans erhält.

Die Konfigurationsangabe *cis* bzw. *trans* in *cis-* bzw. *trans*-1,3-Diaminocyclohexan bezieht sich auf die relative Anordnung der Aminogruppen bezüglich der Cyclohexan-Ringebene. Es ist ersichtlich, dass die Zahl der Stereoisomere höher ist, wenn neben den beiden Aminogruppen weitere Substituenten am Cyclohexanring vorliegen. Für die Zwecke der vorliegenden Erfindung werden diese Stereoisomere zwei Gruppen zugeordnet, nämlich einer Gruppe, in der die Aminogruppen *cis* zueinander vorliegen, und einer Gruppe, in der die Aminogruppen *trans* zueinander vorliegen.

In bestimmten Ausführungsformen trägt das 1,3-Diaminocyclohexan wenigstens eine C₁-C₆-Alkylgruppe in α-Position zu wenigstens einer Aminogruppe. Ein bevorzugtes 1,3-Diaminocyclohexan ist 4-Methylcyclohexan-1,3-diamin, 2-Methylcyclohexan-1,3-diamin oder ein Gemisch davon. Besonders bevorzugt enthält das Gemisch 50 bis 95 Gew.-% 4-Methylcyclohexan-1,3-diamin und 5 bis 50 Gew.-% 2-Methylcyclohexan-1,3-diamin, bezogen auf die Gesamtmenge an 1,3-Diaminocyclohexan.

Die Erfindung betrifft auch 4-Methylcyclohexan-1,3-diamin, 2-Methylcyclohexan-1,3-diamin oder ein Gemisch davon, mit einem *trans-*Gehalt von 99 Mol-% oder mehr, bezogen auf die Summe von 4-Methylcyclohexan-1,3-diamin und 2-Methylcyclohexan-1,3-diamin.

Es wurde gefunden, dass in einem Gemisch von *cis-* und *trans*-1,3-Diaminocyclo-hexanen bei Einwirkung von Kohlendioxid oder einem reaktiven Kohlensäurederivat selektiv das *cis*-Stereoisomer zum Harnstoff, nämlich dem 2,4-Diazabicyclo-[3.3.1]nonan-3-on, umgesetzt wird. Die Reaktion des trans-Stereoisomers mit Kohlendioxid oder einem reaktiven Kohlensäurederivat bleibt auf der Stufe des Carbamats stehen; ein intramolekularer Ringschluss erfolgt nicht. Im Zuge der Aufarbeitung, z.B. durch Abstrippen von CO₂ und/oder thermisch, kann das Carbamat des *trans-*Stereoisomers zum freien *trans*-1,3-Diaminocyclohexan rückgespalten werden.

Das Harnstoffreaktionsprodukt des *cis*-4-Methylcyclohexan-1,3-diamins ist nachstehend veranschaulicht:

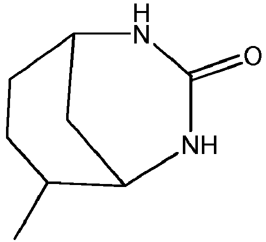

Das Harnstoffderivat kann dann leicht vom trans-1,3-Diaminocyclohexan abgetrennt werden, z. B. durch Fällung, Kristallisation oder Destillation. In der Regel ist das Harnstoffreaktionsprodukt in wässrigen Lösungen schwer löslich und fällt aus. Durch Abkühlen kann die Fällung vervollständigt werden. Vorzugsweise umfasst die Abtrennung ein mechanisches Trennverfahren, wie Filtration, Sedimentation und/oder Zentrifugation, worunter eine Filtration bevorzugt ist.

Das reaktive Kohlensäurederivat ist z.B. ausgewählt unter Harnstoff, Kohlensäureestern, wie Dialkylcarbonaten,wie Dimethylcarbonat, Diethylcarbonat; Alkylencarbonaten, wie Ethylencarbonat, Propylencarbonat; und Phosgen.

Die Bedingungen der Umsetzung hängen von der Reaktivität des reaktiven Kohlensäurederivats ab. In der Regel erfolgt die Umsetzung bei erhöhter Temperatur, z. B. 25 bis 300 °C, vorzugsweise 25 bis 200 °C. Die Umsetzung kann in Substanz oder in Gegenwart eines Lösungsmittels erfolgen.

Kohlendioxid ist das bevorzugte Reagens. Die Umsetzung mit CO₂ kann einstufig bei erhöhtem Druck durchgeführt werden, z. B. 1,5 bis 250 bar, vorzugsweise 5 bis 200 bar, besonders bevorzugt 10 bis 60 bar und erhöhter Temperatur, z. B. 25 bis 300 °C, vorzugsweise 100 bis 250 °C. Sie kann auch zweistufig durchgeführt werden, z.B. in einer Beladungsstufe bei niedrigem Druck und niedriger Temperatur oder hohem Druck und niedriger Temperatur und einer Temperstufe bei höherer Temperatur und variablem Druck. Zwischen diesen Stufen kann überschüssiges CO₂ abgelassen und zurückgeführt werden. Die Reaktionsdauer bzw. Verweilzeit bei kontinuierlich durchgeführtem Verfahren kann von 0,1 bis 24 h pro Stufe betragen.

Zur Umsetzung mit CO₂ liegt das Gemisch von *cis-* und *trans*-1,3-Diaminocyclohexanen im Allgemeinen gelöst vor, muss aber nicht zwangsläufig mit einer anderen Substanz verdünnt sein. Geeignete Lösungsmittel sind Wasser, Alkohole, Ether. Vorzugsweise erfolgt die Umsetzung mit CO₂ in wässriger Lösung. Hierzu kann man eine mit Kohlendioxid beladene wässrige Lösung von *cis-* und *trans*-1,3-Diaminocyclohexanen unter Eigendruck in einem Druckgefäß erwärmen. Der CO₂-Beladungsgrad (ausgedrückt als Mol(CO₂) pro Mol(1,3-Diaminocyclohexan)) beträgt vorzugsweise wenigstens 0,5, insbesondere wenigstens 1,0.

Die Umsetzung kann auch kontinuierlich oder halbkontinuierlich durchgeführt werden. Geeignet ist eine Reaktorkaskade mit einem Vorsättigungreaktor und einem Carboxylierungsreaktor. Im Vorsättigungsreaktor wird eine Lösung von *cis-* und *trans*-1,3-Diaminocyclohexan durch Einspeisen von Kohlendioxid mit Kohlendioxid vorgesättigt, vorzugsweise bei einer Temperatur von 10 bis 50 °C, z. B. Zimmertemperatur. Die CO₂-beladene Lösung wird dann im Carboxylierungsreaktor bei erhöhtem Druck und erhöhter Temperatur umgesetzt, wobei selektiv das cis-Stereoisomer zum Harnstoff reagiert.

In einer bevorzugten Ausführungsform umfasst das Verfahren außerdem eine Anreicherung von *trans*-1,3-Diaminocyclohexan durch Extraktivdestillation in Gegenwart eines Extraktionsmittels. Die Anreicherung durch Extraktivdestillation erfolgt vorzugsweise vor der erfindungsgemäßen Umsetzung mit Kohlendioxid oder einem reaktiven Kohlensäurederivat. D.h. ein bei der Extraktivdestillation erhaltenes *trans-*angereichertes 1,3-Diaminocyclohexan-Gemisch wird erfindungsgemäß mit Kohlendioxid oder einem reaktiven Kohlensäurederivat umgesetzt, wodurch eine weitere *trans-*Anreicherung erfolgt und schließlich im Wesentlichen reines *trans*-1,3-Diaminocyclohexan erhalten werden kann.

Das 1,3-Diaminocyclohexan-Ausgangsgemisch der Extraktivdestillation hat bevorzugt einen Anteil an trans-Isomeren von 5 bis 60 Gew.-% und einen Anteil an *cis*-Isomeren von 40 bis 95 Gew.-%, besonders bevorzugt einen Anteil an trans-Isomeren von 10 bis 55 Gew.% und einen Anteil an *cis*-Isomeren von 45 bis 90 Gew.-% sowie ganz besonders bevorzugt einen Anteil an trans-Isomeren von 20 bis 50 Gew.-%und einen Anteil an *cis*-Isomeren von 50 bis 80 Gew.-%, jeweils bezogen auf die im Ausgangsgemisch enthaltende Gesamtmenge an 1,3-Diaminocyclohexan. Im trans-angereicherten 1,3-Diaminocyclohexan-Gemisch ist der Anteil an trans-Isomeren, bezogen auf die im Gemisch enthaltene Gesamtmenge an 1,3-Diaminocyclohexan, höher als der Anteil an *trans*-Isomeren im Ausgangsgemisch. Das *trans*-angereicherte 1,3-Diaminocyclohexan-Gemisch hat bevorzugt einen Anteil an *trans*-Isomeren von mehr als 60 Gew.-% und einen Anteil an *cis*-Isomeren von weniger als 40 Gew.-%, besonders bevorzugt einen Anteil an *trans*-Isomeren von 60 bis 80 Gew.% und einen Anteil an *cis*-Isomeren von 40 bis 20 Gew.-% sowie ganz besonders bevorzugt einen Anteil an trans-Isomeren von 90 bis 99,99 Gew.-%und einen Anteil an *cis*-Isomeren von 10 bis 0,01 Gew.-%, jeweils bezogen auf die im trans-angereicherten 1,3-Diaminocyclohexan-Gemisch enthaltende Gesamtmenge an 1,3-Diaminocyclohexan.

Als Extraktionsmittel kommen Verbindungen in Betracht, die mit den *cis*-Isomeren des 1,3-Diaminocyclohexans ein Schwersiederazeotrop bilden Das Extraktionsmittel weist geeigneterweise einen Siedepunkt auf, der mindestens 5 °C über dem Siedepunkt des am niedrigsten siedenden 1,3-Diaminocyclohexan-Isomers im Ausgangsgemisch liegt. Das Extraktionsmittel liegt bei der Sumpftemperatur der Extraktivdestillation flüssig vor. Es enthält wenigstens zwei unter Hydroxy- und Aminogruppen ausgewählte funktionelle Gruppen im Molekül. Geeignete Extraktionsmittel unter sind unter Polyolen, Aminoalkoholen und Polyaminen ausgewählt.

Zu geeigneten Polyolen zählen Ethylenglycol, 1,2-Propandiol, 2-Methylpropan-1,3-diol, 1,2-Butandiol, 2,3-Butandiol, 2-Methyl-butan-1,2-diol, 3-Methylbutan-1,2-diol, 3-Methyl-1,3-butandiol, 1,2-Pentandiol, 1,3-Pentandiol, 2,4-Pentandiol, 2,3-Pentandiol, 1,2-Hexandiol, *cis*-1,2-Cyclopentandiol, *trans*-1,2-Cyclopentandiol, *cis*-1,2-Cyclohexandiol, *trans-1,2-Cyclohexandiol,* 1,3-Propandiol, 2-Methyl-1,3-propandiol, 2,2,-Dimethyl-1,3-propandiol (Neopentylglycol), 1,3-Butandiol, 1,2-Pentandiol, 2,4-Pentandiol, 1,5-Pentandiol, 1,3-Hexandiol, 2,4-Hexandiol, 1,3-Cyclobutandiol, 1,3-Cyclopentandiol, 1,3-Cyclohexandiol, *cis-* und *trans*-1,4-Butendiol, 1,4-Butandiol, 2,3-Dimethyl-1,4-Butandiol, 2,2-Dimethyl-1,4-butandiol, 1,4-Pentandiol, 2,3-Dimethyl-1,5-pentandiol, 1,4-Hexandiol, 1,4-Cyclohexandiol, 1,3,6-Hexantriol, 1,2,3-Hexantriol, 1,2,6-Hexantriol, Glycerin, Diglycerin, Sorbitol, Pentaerythrit, Diethylenglykol, Triethylenglycol, Dipropylenglycol.

Zu geeigneten Aminoalkoholen zählen Diethanolamin, N-Methyldiethanolamin, N-Propyldiethanolamin, N-Butyldiethanolamin, Triethanolamin, N-Ethylpropanolamin, N-Propylethanolamin, N,N-Dipropylethanolamin, N-Butylethanolamin, N,N-Dibutyl-ethanolamin, Propanolamin, Dipropanolamin, N-Methyldipropanolamin, N-Propyldipropanolamin, N-Butyldipropanolamin, Tripropanolamin, Diisopropanolamin, N-Methyl-diisopropanolamin, Triisopropanolamin, N-2-Methylaminopropanol, 4-Amino-1-butanol, 4-(2-Hydroxyethyl)morpholin, Pentanolamin, Hydroxyethylpiperazin, N-(2-Hydroxyethyl)anilin, N,N-Di-(2-hydroxyethyl)anilin, 3-Amino-1-propanol.

Zu geeigneten Polyaminen zählen 2-(Diisopropylamino)ethylamin, 3-(Cyclohexyl-amino)proplyamin, Dipropylentriamin, Triethylentetramin, Pentamethyldiethylentriamin, 3-(2-Aminoethylamino)propylamin, Diethylentriamin, Isophorondiamin.

Ganz besonders bevorzugt sind Glycerin, 1,3-Propandiol, 1,4-Butandiol, cis-1,4-Butendiol, Triethylenglykol, Diglycerin, 1,5-Pentandiol, 4-(2-Hydroxyethyl)morpholin, N-(2-Hydroxyethyl)-anilin, Triethanolamin, N-Methyldiethanolamin. Ganz besonders bevorzugt sind insbesondere 1,3-Propandiol und 1,4-Butandiol.

Bei der Extraktivdestillation kann trans-angereichertes 1,3-Diaminocyclohexan über Kopf erhalten werden. Besonders bevorzugt werden das Ausgangsgemisch und das Extraktionsmittel getrennt voneinander einer Destillationskolonne zugeführt. Ganz besonders bevorzugt erfolgt die Zuführung des Extraktionsmittels oberhalb der Zuführung des Ausgangsgemischs. Die Zuführung des Extraktionsmittels und des Ausgangsgemischs kann sowohl flüssig, gasförmig, als auch flüssig siedend erfolgen.

*trans*-angereichertes 1,3-Diaminocyclohexan wird üblicherweise am Kopf der Kolonne als Destillat oder als Seitenstrom abgezogen. Wird das *trans*-angereicherte 1,3-Diaminocyclohexan im Seitenstrom abgezogen, so befindet sich die Seitenabzugsstelle bevorzugt mindestens 1, besonders bevorzugt mindestens 5 theoretische Stufen über der Zulaufstelle des Extraktionsmittels.

Der Anteil an Extraktionsmittels im *trans*-angereicherten 1,3-Diaminocyclohexan ist üblicherweise dann gering, wenn eine Kolonne mit einer hohen Trennleistung eingesetzt wird und ausreichend viele Trennstufen zwischen der Zuführung des Extraktionsmittels und dem Abzug des trans-angereicherten 1,3-Diaminocyclohexans (im Destillat und/oder Seitenabzug) liegen. Eine weitere Möglichkeit zur Verringerung des Anteils an Extraktionsmittels im *trans*-angereicherten 1,3-Diaminocyclohexan besteht darin, einen zweiten Destillationsschritt durchzuführen.

Die Erfindung wird durch die nachfolgenden Beispiele näher veranschaulicht:
Es werden folgende Abkürzungen verwendet:
   MDACH: 4-Methylcyclohexan-1,3-diamin

### Beispiel 1: Umsetzung von cis-MDACH in Harnstoff in Anwesenheit von CO₂

Wässrige Mischungen bestehend aus 10 Gew% MDACH (60% *cis*-Isomere/40% *trans*-Isomere) wurden angesetzt. Jeweils 8 ml einer unbeladenen sowie mit 15 Nm³/t CO₂ beladenen Mischung wurden mit Stickstoff inertisiert und in 10 ml Edelstahl (1.4571) Autoklaven eingefüllt. Anschließend wurden die Autoklaven verschlossen, in einem Ölbad auf 160 °C aufgeheizt und dort über 5 Tage bei 160°C temperiert. Nach Abkühlen der Autoklaven wurden diese geöffnet und beprobt. Hierzu wurde zunächst das restliche CO₂ aus der Lösung mit N₂ gestrippt und anschließend die nahezu sauergasfreien Lösungen im Gaschromatographen auf den Amingehalt und gebildete Nebenkomponenten hin untersucht. Die Zusammensetzung der Proben ist in Tabelle 1 zusammengefasst.

**Tabelle 1: Zusammensetzung 10% MDACH Lösungen (GC-Flächen bezogen, ohne Wasser). A: unbehandelt, B: 125 h bei 160°C ohne CO₂, C: 125 h bei 160°C mit CO₂.**

| Probenbezeichnung | A | B | C |
|---|---|---|---|
| Summe MDACH *cis* Isomere | 61,65 | 61,47 | 34,18 |
| Summe MDACH *trans* Isomere | 38,35 | 38,53 | 38,17 |
| Harnstoff | 0 | 0 | 27,65 |

Beispiel 2: Vergleich verschiedener MDACH Konzentrationen bei unterschiedlichen CO₂ Beladungen.

Durch Extraktivdestillation mit 1,3-Propylenglycol als Extraktionsmittel wurde trans-angereichertes MDACH (20% *cis*-Isomere/ 80% trans-Isomere) erhalten. Wässrige Mischungen bestehend aus 10, 30 und 50 Gew% MDACH (20% *cis*-Isomere/ 80% trans-Isomere) wurden angesetzt. Jeweils 8 ml Lösung mit unterschiedlichen Beladungen (Details siehe Tabelle 2) wurden mit Stickstoff inertisiert und in 10 ml Edelstahl (1.4571) Autoklaven eingefüllt. Anschließend wurden die Autoklaven verschlossen, in einem Ölbad auf 160 °C aufgeheizt und dort über 5 Tage bei 160 °C temperiert. Nach Abkühlen der Autoklaven wurden diese geöffnet und beprobt. Hierzu wurde zunächst das restliche CO₂ aus den Lösungen mit N₂ gestrippt und anschließend die nahezu sauergasfreien Lösungen im Gaschromatographen auf den Amingehalt und gebildete Nebenkomponenten hin untersucht. Die Zusammensetzung der Proben ist in Tabelle 2 zusammengefasst.

**Tabelle 2: Zusammensetzung versch. MDACH Lösungen (GC-Flächen bezogen, ohne Wasser), mit unterschiedlichen initialen MDACH Konzentrationen und CO₂-Beladungen.**

| | A | B | C | D | C |
|---|---|---|---|---|---|
| Gehalt MDACH in Wasser [Gew%] | 10 | 10 | 10 | 30 | 50 |
| 125 h bei 160 °C | nein | ja | ja | ja | ja |
| CO₂-Beladung [Nm³/t] | 0 | 15 | 27 | 69 | 100 |
| Summe MDACH *cis* Isomere | 19,23 | 4,92 | 1,23 | 1,51 | 0,11 |
| Summe MDACH *trans* Isomere | 80,61 | 79,55 | 79,06 | 71,81 | 64,63 |
| Harnstoff | 0 | 15,53 | 19,71 | 26,68 | 35,26 |
| *trans-Isomer bezogen auf cis- und trans-MDACH* | 80,7% | 94,2% | 98,4% | 97,9% | 99,8% |

### Beispiel 3: Vergleich verschiedener Reaktionszeiten

Wässrige Mischungen bestehend aus 10 Gew% MDACH (60% *cis*-Isomere/ 40% trans-Isomere) wurden angesetzt. Jeweils 8 ml voll beladener Lösung (27 Nm³/t) wurden mit Stickstoff inertisiert und in 10 ml Edelstahl (1.4571) Autoklaven eingefüllt. Anschließend wurden die Autoklaven verschlossen, in einem Ölbad auf 160°C aufgeheizt und dort über 2, 4 und 6 Tage bei 160°C temperiert. Nach Abkühlen der Autoklaven wurden diese geöffnet und beprobt. Hierzu wurde zunächst das restliche CO₂ aus den Lösungen mit N₂ gestrippt und anschließend die nahezu sauergasfreien Lösungen im Gaschromatographen auf den Amingehalt und gebildete Nebenkomponenten hin untersucht. Die Zusammensetzung der Proben ist in Tabelle 3 zusammengefasst.

**Tabelle 3: Zusammensetzung von MDACH Lösungen (GC-Flächen bezogen, ohne Wasser), nach unterschiedlichen Versuchszeiten, T =160°C, Initiale CO₂-Beladung: 27 Nm³/t.**

| | A | B | C | D |
|---|---|---|---|---|
| Dauer Begasung mit CO₂ [h] | 0 | 48 | 96 | 144 |
| Summe MDACH *cis* Isomere | 62,13 | 40,60 | 26,42 | 16,80 |
| Summe MDACH *trans* Isomere | 37,87 | 38,15 | 38,11 | 36,93 |
| Harnstoff | 0 | 21,25 | 35,46 | 46,27 |

### Beispiel 4:

In einem 160 mL Autoklaven aus Edelstahl mit einem Schrägblattrührer wurden 12 g MDACH in 28 g Wasser gelöst. Der Autoklav wurde verschlossen und auf 25°C temperiert. Unter Rühren mit 500 Umdrehungen/Min. wurden 60 bar CO₂ aufgepresst und der Druck konstant gehalten, indem weiteres CO₂ zudosiert wurde, wenn der Druck abnahm. Nach einer Stunde Rühren wurde der Druck auf 2 bar entspannt. Anschließend wurde die Temperatur unter Rühren bei 200 U/min auf 180°C erhöht und nach Erreichen dieser Temperatur wurde 20h bei 500 U/min weitergerührt. Nach vollendeter Reaktionszeit wurde auf Raumtemperatur abgekühlt und entspannt. Der teilweise feste Inhalt wurde in eine Flasche überführt. Diese Prozedur wurde zwei Mal wiederholt, dann wurden die vereinigten Reaktionsausträge aus den drei Versuchen über eine Filternutsche abgesaugt und der Filterkuchen mit 2x20 mL Eiswasser gewaschen. Nach Trocknung des so erhaltenen wasserfeuchten Filterkuchens im Vakuum für 17 h wurden 16,3 g Harnstoff gewonnen. Der Gehalt an Harnstoff laut GC-Analytik betrug 98,1%, der Rest bestand aus MDACH.

### Beispiel 5:

In einem 160 mL Autoklaven aus Edelstahl mit einem Schrägblattrührer wurden 12 g MDACH in 28 g Wasser gelöst. Der Autoklav wurde verschlossen und auf 25°C temperiert. Unter Rühren mit 500 Umdrehungen/Min. wurden 60 bar CO₂ aufgepresst und der Druck konstant gehalten, indem weiteres CO₂ zudosiert wurde, wenn der Druck abnahm. Nach einer Stunde Rühren wurde der Druck auf 2 bar entspannt. Anschließend wurde die Temperatur unter Rühren bei 200 U/min auf 160°C erhöht und nach Erreichen dieser Temperatur wurde 14h bei 500 U/min weitergerührt. Nach dieser Zeit wurde eine Probe per GC analysiert. Das *cis*/*trans* Verhältnis betrug 4,1:95,9. Der Autoklaveninhalt wurde noch einmal behandelt wie oben beschrieben und wieder 14 h getempert. Danach betrug das Verhältnis von *cis* zu *trans*-Isomer 0,2:99,8. Die Mischung der Produkte bestand aus 80% MCDA, 15,3% cis-Harnstoff und 4,7% sonstigen Produkten, deren Identität nicht aufgeklärt worden war.

## Patentansprüche

1. Verfahren zur Diskriminierung von *cis-* und *trans*-1,3-Diaminocyclohexanen, wobei man ein Gemisch von *cis-* und *trans*-1,3-Diaminocyclohexanen mit Kohlendioxid oder einem reaktiven Kohlensäurederivat umsetzt und selektiv den Harnstoff des *cis*1,3-Diaminocyclohexans erhält.

2. Verfahren nach Anspruch 1, wobei das reaktive Kohlensäurederivat ausgewählt ist unter Harnstoff, Kohlensäureestern und Phosgen.

3. Verfahren nach Anspruch 1 oder 2, wobei man den Harnstoff des *cis*-1,3-Diaminocyclohexans abtrennt.

4. Verfahren nach Anspruch 3, wobei die Abtrennung ein mechanisches Trennverfahren umfasst.

5. Verfahren nach einem der vorhergehenden Ansprüche, wobei die Umsetzung mit Kohlendioxid oder dem reaktiven Kohlensäurederivat in wässriger Lösung erfolgt.

6. Verfahren nach Anspruch 5, wobei man eine mit Kohlendioxid beladene wässrige Lösung von *cis-* und *trans*-1,3-Diaminocyclohexanen unter Eigendruck in einem Druckgefäß erwärmt.

7. Verfahren nach einem der vorhergehenden Ansprüche, außerdem umfassend eine Anreicherung von *trans*-1,3-Diaminocyclohexan durch Extraktivdestillation in Gegenwart eines Extraktionsmittels.

8. Verfahren nach Anspruch 7, wobei das Extraktionsmittel unter Polyolen, Aminoalkoholen und Polyaminen ausgewählt ist.

9. Verfahren nach einem der vorhergehenden Ansprüche, wobei das 1,3-Diaminocyclohexan wenigstens eine C₁-C₆-Alkylgruppe in α-Position zu wenigstens einer Aminogruppe trägt.

10. Verfahren nach Anspruch 9, wobei das 1,3-Diaminocyclohexan 4-Methylcyclohexan-1,3-diamin, 2-Methylcyclohexan-1,3-diamin oder ein Gemisch davon ist.

11. 4-Methylcyclohexan-1,3-diamin, 2-Methylcyclohexan-1,3-diamin oder Gemisch davon, mit einem *trans*-Gehalt von 99 Mol-% oder mehr, bezogen auf die Summe von 4-Methylcyclohexan-1,3-diamin und 2-Methylcyclohexan-1,3-diamin.
